# EUROPEAN PATENT SPECIFICATION

(11) **EP 1 983 984 B1**
(45) Date of publication and mention of the grant of the patent: **07.03.2018**
(21) Application number: 07711417.1
(22) Date of filing: 31.01.2007
(51) Int. Cl.: A61K 31/436, A61K 31/365, A61K 31/351, A61P 35/00, A61P 25/00

(54) **TUBEROUS SCLEROSIS TREATMENT**
BEHANDLUNG VON TUBERÖSER SKLEROSE
TRAITEMENT DE LA SCLEROSE TUBEREUSE

(30) Priority: 02.02.2006 GB 0602123; 22.02.2006 GB 0603568; 07.03.2006 GB 0604593; 22.03.2006 GB 0605760; 16.05.2006 GB 0609698
(43) Date of publication of application: 29.10.2008
(60) Divisional application: 18151704.6
(73) Proprietor: Novartis AG, 4056 Basel (CH)
(72) Inventor: BERG, William, New City, NY 10956 (US); BENEDETTO, John, Morristown, NJ 07960 (US); ELMROTH, Ingrid, CH-4125 Riehen (CH); LANE, Heidi, CH-4105 Biel-Benken (CH); LEBWOHL, David, Madison, NJ 07940 (US); SELLERS, William, Chestnut Hill, MA 02467 (US); STUMM, Michael, CH-4125 Riehen (CH)
(74) Representative: Graff, Alan
(86) International application number: PCT/EP2007/000818
(87) International publication number: WO 2007/088034

(56) References cited:
- WO-A-02/11742
- WO-A-2004/073614
- WO-A-2005/052187
- US-A1- 2006 160 837
- FRANZ D N ET AL: "Rapamycin causes regression of astrocytomas in tuberous sclerosis complex" ANNALS OF NEUROLOGY 2006 UNITED STATES, vol. 59, no. 3, 1 February 2006 (2006-02-01), pages 490-498, XP002430704 ISSN: 0364-5134
- MAK BALDWIN C ET AL: "The tuberous sclerosis complex genes in tumor development." CANCER INVESTIGATION 2004, vol. 22, no. 4, 2004, pages 588-603, XP002430705 ISSN: 0735-7907
- WIENECKE RALF ET AL: "Antitumoral activity of rapamycin in renal angiomyolipoma associated with tuberous sclerosis complex." AMERICAN JOURNAL OF KIDNEY DISEASES : THE OFFICIAL JOURNAL OF THE NATIONAL KIDNEY FOUNDATION SEP 2006, vol. 48, no. 3, September 2006 (2006-09), pages e27-e29, XP002430706 ISSN: 1523-6838
- JOHNSON S R: "Lymphangioleiomyomatosis" EUROPEAN RESPIRATORY JOURNAL 2006 SWITZERLAND, vol. 27, no. 5, 2006, pages 1056-1065, XP002430707 ISSN: 0903-1936
- GEMMILL R M ET AL: "Synergistic Growth by Iressa and Rapamycin is modulated by VHL mutation in renal cell carcinoma" BRITISH JOURNAL OF CANCER, LONDON, GB, vol. 92, June 2005 (2005-06), pages 2266-2277, XP003001047 ISSN: 0007-0920
- DASGUPTA BIPLAB ET AL: "Proteomic analysis reveals hyperactivation of the mammalian target of rapamycin pathway in neurofibromatosis 1-associated human and mouse brain tumors" CANCER RESEARCH, vol. 65, no. 7, April 2005 (2005-04), pages 2755-2760, XP002430708 ISSN: 0008-5472
- SARBASSOV ET AL: "Growing roles for the mTOR pathway", CURRENT OPINION IN CELL BIOLOGY, CURRENT SCIENCE, LONDON, GB, vol. 17, no. 6, 1 December 2005 (2005-12-01), pages 596-603, XP005136507, ISSN: 0955-0674, DOI: 10.1016/J.CEB.2005.09.009

## Description

The present application relates to methods for treating and preventing disorders mediated via the Tuberous Sclerosis Complex (TSC), e.g. tuberous sclerosis.

Neurocutaneous disorders as used herein include disorders mediated via the Tuberous Sclerosis Complex (TSC), e.g. tuberous sclerosis and related disorders and neurofibromatosis type 1 and related disorders.
Neurofibromatosis type 1 (NF1) and tuberous sclerosis complex (TSC) represent two neurocutaneous disorders in which affected individuals develop tumors at an increased frequency.

TSC is an autosomal dominant disorder characterized by widespread benign hamartomas, epilepsy, mental retardation, and autism. TSC is linked to mutations in the tumor suppressor genes, TSC1 and TSC2. Mutation in either of these two genes leads to the clinical manifestations of TSC. Common clinical symptoms include seizures, mental retardation, autism, kidney failure, facial angiofibromas and cardial Rhabdomyomas, and, in addition, many affected individuals have cyst-like areas within certain skeletal regions, particularly bones of the fingers and toes (phalanges). Characteristic skin lesions include sharply defined areas of decreased skin coloration (hypopigmentation) that may develop during infancy and relatively small reddish nodules that may appear on the cheeks and nose beginning at approximately age four. These reddish lesions eventually enlarge, blend together (coalesce), and develop a wart-like appearance (sebaceous adenomas). Additional skin lesions may also develop, including flat, "coffee- colored" areas of increased skin pigmentation(cafe-au-lait spots); benign, fibrous nodules (fibromas) arising around or beneath the nails; or rough, elevated, "knobby" lesions (shagreen patches) on the lower back.
While many of the features of TSC are neurological in nature, renal dysfunction is a common characteristic of the disease. Approximately 70-80% of TSC patients develop renal angiomyolipomas (AMLs). AMLs are heterogeneous, benign tumors composed of three distinct cell types including smooth muscle, blood vessel, and adipose cells. TSC patients also present with evidence of a devastating form of lung disease called Lymphangioleiomyomatosis (LAM). LAM is a unique and rare cystic pulmonary disease (lung) disease that afflicts predominately premenopausal women. Over time, the muscle cells block the flow of air, blood, and lymph to and from the lungs, preventing the lungs from providing oxygen to the rest of the body. Kidney tumors that are often asymptomatic may also be found in patients with LAM, e.g. clinical symptoms are dysapnea, chronic cough, wheezing, pneumothorax, and chest pain. These symptoms occur and worsen as LAM cells migrate into the lung, causing cystic parenchymal destruction and progressive respiratory failure. LAM can occur as an independent condition (sporadic LAM) or as a secondary condition of TSC (TSC-LAM). AMLs are symptomatic of both LAM (50% of patients presenting) and TSC (70% of patients presenting), and there are no radiological, morphological or genetic differences between AMLs from the two disorder.
It is reported that tuberous sclerosis gene mutations are a cause of lymphyangioleiomyomatosis (LAM). The mutations were found in the angiomyolipoma cells and LAM cells from four women with LAM. The mutations were not present in normal lung, kidney or blood cells, indicating that these women with LAM do not have the inherited disease, tuberous sclerosis. Identifying this genetic link between tuberous sclerosis and sporadic LAM is an important step in LAM research (see e.g. "Mutations in the tuberous sclerosis complex gene TSC2 are a cause of sporatic lymphyangioleiomyomatosis"; Carsillo, Astrinidis and Henske; PNAS 2000 97:6085-90).
A part of TSC patients will develop subependymal giant cell astrocytomas (SEGAs), which are slowly progresing tumors that are typically asymptomatic until ventricular obstruction occurs, resulting in life threatening acute hydrocephalus. Due to the deep location of these tumors, surgical resection is difficult and often associated with significant morbidity.

Surprisingly 40-O-(2-hydroxyethyl)-rapamycin has been found to be effective in tuberous sclerosis and such compound is prone for the treatment of neurocutaneous disorders in which affected individuals develop tumors at an increased frequency, such as tuberous sclerosis, including related disorders.

In one aspect the present invention provides
1.1 A compound of formula 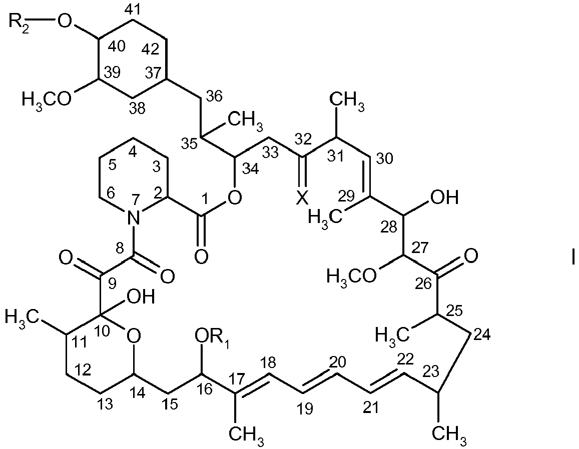 e.g. including a compound of formula 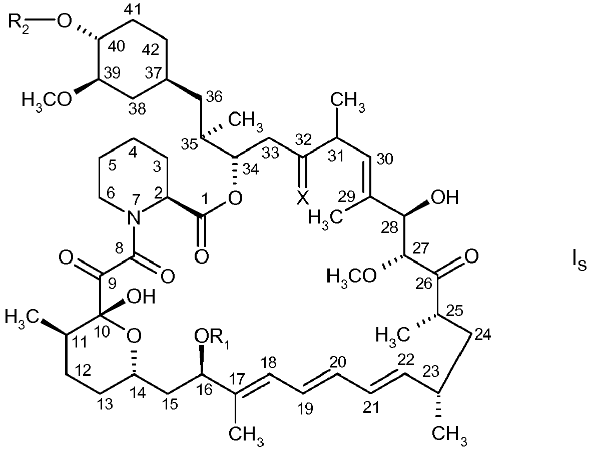 wherein
   R₁ is CH₃,
   R₂ is -CH₂-CH₂-OH,
      and
   X is = O,
   for use in the treatment of disorders mediated via the Tuberous Sclerosis Complex, wherein the disorders are renal angiomyolipomas (AML), lymphangioleiomyomatosis (LAM), and/or subependymal giant cell astrocytomas (SEGAs); and/or for use in the treatment of symptoms associated with disorders mediated via the Tuberous Sclerosis Complex, wherein the symptoms are seizures, wherein 40-O-(2-hydroxyethyl)-rapamycin is administered orally in dosages from 2.5 mg up to 15 mg. Specific embodiments are provided as set forth below in the dependent claims.

The present invention also provides a pharmaceutical composition comprising 40-O-(2-hydroxyethyl)-rapamycin in association with at least one pharmaceutically acceptable excipient, for use in the treatment according to the aspects above.

The present invention also discloses 40-O-(2-hydroxyethyl)-rapamycin for use in the treatment of neurocutaneous disorders.

The present invention further provides
1.2 A compound of formula I wherein R₁, R₂ and X are as defined above,
   for use in the treatment of disorders mediated via the Tuberous Sclerosis Complex, for inducing regression of disorders mediated via the Tuberous Sclerosis Complex, for treating symptoms associated with disorders mediated via the Tuberous Sclerosis Complex, for the treatment of disorders associated with disorders mediated via the Tuberous Sclerosis Complex, and/or for inhibiting or controlling disorders mediated via the Tuberous Sclerosis Complex,
   e.g. wherein disorders mediated via the Tuberous Sclerosis Complex include tuberous sclerosis, renal angiomyolipomas (ALM), lymphangioleiomyomatosis (LAM), subependymal, and/or giant cell astrocytomas (SEGAs).

40-O-(2-hydroxyethyl)-rapamycin is also known under the name everolimus.

40-O-(2-hydroxyethyl)-rapamycin is also designated herein as "TSC-compound of (according to) the present invention".

The present invention provides
1.3 40-O-(2-hydroxyethyl)-rapamycin for use in the treatment of disorders mediated via the Tuberous Sclerosis Complex, comprising administering to a subject in need thereof a therapeutical effective amount of a TSC-compound of the present invention.
1.4 40-O-(2-hydroxyethyl)-rapamycin for use in a method of inducing regression of disorders mediated via the Tuberous Sclerosis Complex, comprising administering to a subject in need thereof a therapeutic effective amount of a TSC-compound of the present invention.
1.5 40-O-(2-hydroxyethyl)-rapamycin for use in the treatment of symptoms associated with disorders mediated via the Tuberous Sclerosis Complex, comprising administering to a subject in need thereof a therapeutically effective amount of a TSC-compound of the present invention.
1.6 40-O-(2-hydroxyethyl)-rapamycin for use in a method of inhibiting or controlling disorders mediated via the Tuberous Sclerosis Complex, comprising administering to a subject in need thereof a therapeutically effective amount of a TSC-compound of the present invention.

The present invention further provides
1.7 A compound for use as indicated under 1.1, 1.2 to 1.6, wherein a compound of the present invention is 40-O-(2-hydroxyethyl)-rapamycin (e.g. herein also designated as "Compound A").

In a preferred aspect the present invention provides
2.1 40-O-(2-hydroxyethyl)-rapamycin for use as indicated under 1.1, 1.7 and 1.2 to 1.6 above, for treating tuberous sclerosis.
2.2 40-O-(2-hydroxyethyl)-rapamycin for use as indicated under 1.1, 1.7 and 1.2 to 1.6 above, for treating renal angiomyolipomas (ALM).
2.3 40-O-(2-hydroxyethyl)-rapamycin for use as indicated under 1.1, 1.7 and 1.2 to 1.6 above, for treating lymphangioleiomyomatosis (LAM).
2.4 40-O-(2-hydroxyethyl)-rapamycin for use as indicated under 1.1, 1.7 and 1.2 to 1.6 above, for treating acute hydrocephalus, e.g. resulting from subependymal giant cell astrocytomas (SEGAs).

The compound of the present invention 40-O-(2-hydroxyethyl)-rapamycin for any use as defined under 1.1, 1.7, 1.2 to 1.6, or 2.1 to 2.4 above, e.g. including preferred aspects as defined above, is preferably used in the form of a pharmaceutical composition.

In another aspect the present invention provides
4.1 A pharmaceutical composition comprising 40-O-(2-hydroxyethyl)-rapamycin in association with at least one pharmaceutically acceptable excipient, e.g. appropriate carrier and/or diluent, e.g. including fillers, binders, disintegrants, flow conditioners, lubricants, sugars or sweeteners, fragrances, preservatives, stabilizers, wetting agents and/or emulsifiers, solubilizers, salts for regulating osmotic pressure and/or buffers; for any use as defined under 1.1, 1.7, 1.2 to 1.6, or 2.1 to 2.4 above, e.g. including preferred aspects as defined above.

The compound of the present invention, 40-O-(2-hydroxyethyl)-rapamycin, may be used for any use and in any pharmaceutical composition as provided by the present invention alone or in combination with one or more, at least one, second drug substance.

The present invention also provides
5.1 A combination of a TSC-compound of the present invention with at least one second drug substance, for any use as defined under 1.1, 1.7, 1.2 to 1.6, or 2.1 to 2.4 above, e.g. including preferred aspects as defined above.
5.2 A pharmaceutical combination comprising a compound of the present invention in combination with at least one second drug substance, for any use as defined under 1.1, 1.7, 1.2 to 1.6, or 2.1 to 2.4 above, e.g. including preferred aspects as defined above.
5.3 A pharmaceutical composition comprising a TSC-compound of the present invention in combination with at least one second drug substance and one or more pharmaceutically acceptable excipient(s), for any use as defined under 1.1, 1.7, 1.2 to 1.6, or 2.1 to 2.4 above, e.g. including preferred aspects as defined above.
5.4 A TSC-compound of the present invention in combination with a second drug substance, for any use as defined under 1.1, 1.7, 1.2 to 1.6, or 2.1 to 2.4 above, e.g. including preferred aspects as defined above.
5.5 A TSC-compound of the present invention in combination with at least one second drug substance for any use as defined under 1.1, 1.7, 1.2 to 1.6, or 2.1 to 2.4 above, e.g. including preferred aspects as defined above.
5.6 A TSC-compound of the present invention for any use as defined under 1.1, 1.7, 1.2 to 1.6, or 2.1 to 2.4 above, e.g. including preferred aspects as defined above, comprising co-administering, concomitantly or in sequence, a therapeutically effective amount of the TSC-compound of the present invention and at least one second drug substance, e.g. in the form of a pharmaceutical combination or composition.
5.7 A TSC-compound of the present invention for use as defined under 5.6 above, wherein a TSC-compound of the present invention is administered intermittently.

Combinations include fixed combinations, in which a compound of the present invention, and at least one second drug substance are in the same formulation; kits, in which a compound of the present invention and at least one second drug substance in separate formulations are provided in the same package, e.g. with instruction for co-administration; and free combinations in which a compound of the present invention and at least one second drug substance are packaged separately, but instruction for concomitant or sequential administration are given;
e.g wherein a compound of the present invention is 40-O-(2-hydroxyethyl)-rapamycin.

The present invention also provides
5.8 A pharmaceutical package comprising a first drug substance which is a TSC-compound of the present invention and at least one second drug substance, beside instructions for combined administration;
5.9 A pharmaceutical package comprising a TSC-compound of the present invention beside instructions for combined administration with at least one second drug substance;
5.10 A pharmaceutical package comprising at least one second drug substance beside instructions for combined administration with a TSC-compound of the present invention;
for any use as defined under 1.1, 1.7, 1.2 to 1.6, or 2.1 to 2.4 above, e.g. including preferred aspects as defined above.

Treatment with combinations according to the present invention may provide improvements compared with single treatment.

The present invention also provides
5.11 A pharmaceutical combination comprising an amount of a TSC-compound of the present invention and an amount of a second drug substance, wherein the amounts are appropriate to produce a synergistic therapeutic effect;
5.12 40-O-(2-hydroxyethyl)-rapamycin for use in the method for improving the therapeutic utility of a TSC-compound of the present invention comprising co-administering, e.g. concomitantly or in sequence, a therapeutically effective amount of a compound of the present invention and a second drug substance.
5.13 40-O-(2-hydroxyethyl)-rapamycin for use in the method for improving the therapeutic utility of a second drug substance comprising co-administering, e.g. concomitantly or in sequence, a therapeutically effective amount of a TSC-compound of the present invention and a second drug substance.
for use as defined under 1.1, 1.7, 1.2 to 1.6, or 2.1 to 2.4 above.

In a pharmaceutical combination as defined under 5.11 to 5.13 above the activity of a TSC-compound of the present invention or a second drug substance may be enhanced compared with single treatment, e.g. combined treatment may result in synergistic effects or may overcome resistance against a TSC-compound of the present invention or a chemotherapeutic agent, e.g. when used as defined under 1.1, 1.7, 1.2 to 1.6, or 2.1 to 2.4 above.

A (pharmaceutical) combination, e.g. composition as indicated under 5.1 to 5.13 comprises
a) a first agent which is a TSC-compound of the present invention and
b) a second drug substance as a co-agent which is a chemotherapeutic agent, e. g. as defined hereinafter or hereinbefore.

Treatment as provided by the present invention includes prophylaxis (prevention). Disorders as used herein include diseases.

For such treatment, the appropriate dosage will, of course, vary depending upon, for example, the chemical nature and the pharmacokinetic data of a compound used, the individual host, the mode of administration and the nature and severity of the conditions being treated. However, in general, for satisfactory results in larger mammals, for example humans, an indicated daily dosage includes a range
- from about 0.0001 g to about 1.5 g, such as 0.001 g to 1.5 g;
- from about 0.001 mg/kg body weight to about 20 mg/kg body weight, such as 0.01 mg/kg body weight to 20 mg/kg body weight,
for example administered in divided doses up to four times a day.

In a use, combination, pharmaceutical combination or pharmaceutical composition provided by the present invention a compound of the present invention, 40-O-(2-hydroxyethyl)-rapamycin, may be administered as appropriate, e.g. in dosages which are known for compounds of the present invention, by any administration route, e.g. enterally, e.g. orally, or parenterally. E.g. everolimus may be administered, e.g. orally, in dosages from 0.1 mg up to 15 mg, such as 0.1 mg to 10 mg. e.g. 0.1 mg, 0.25 mg, 0.5 mg, 0.75 mg, 1 mg, 2.5 mg, 5 mg, or 10 mg, more preferably from 0.5 mg to 10 mg, e.g. in the form of (dispersible) tablets; e.g. comprising everolimus in the form of a solid dispersion; e.g. a weekly dosage may include up to 70 mg, e.g. 10 to 70 mg, such as 30 to 50 mg, e.g. depending on the disease being treated.

A second drug substance may be administered in combination therapy as appropriate, e.g. according to a method as conventional, e.g. analogously to administration indications given for a specified drug for single treatment.

A second drug substance as used herein may be administered by any conventional route, for example enterally, e.g. including nasal, buccal, rectal, oral, administration; parenterally, e.g. including intravenous, intraarterial, intramuscular, intracardiac, subcutanous, intraosseous infusion, transdermal (diffusion through the intact skin), transmucosal (diffusion through a mucous membrane), inhalational administration; topically; e.g. including epicutaneous, intranasal, intratracheal administration; intraperitoneal (infusion or injection into the peritoneal cavity); epidural (peridural) (injection or infusion into the epidural space); intrathecal (injection or infusion into the cerebrospinal fluid); intravitreal (administration via the eye); or via medical devices, e.g. for local delivery, e.g. stents;
e.g. in form of coated or uncoated tablets, capsules, (injectable) solutions, infusion solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.

A second drug substance as used herein may be administered in the form of a pharmaceutically acceptable salt, or in free form; optionally in the form of a solvate. Pharmaceutical compositions according to the present invention may be manufactured according, e.g. analogously, to a method as conventional, e.g. by mixing, granulating, coating, dissolving or lyophilizing processes. Unit dosage forms may contain, for example, from about 0.1 mg to about 1500 mg, such as 1 mg to about 1000 mg.
Pharmaceutical compositions comprising a combination of the present invention and pharmaceutical compositions comprising a second drug substance as described herein, may be provided as appropriate, e.g. according, e.g. analogously, to a method as conventional, or as described herein for a pharmaceutical composition of the present invention.

Use in a method for treating diseases mediated via the Tuberous Sclerosis Complex includes diseases which are the result of a defective Tsc pathway in a subject.
A "defective Tsc pathway" includes regulation of the Tsc pathway that results in a biological effect that causes adverse effects on a cell or tissue within the Tsc pathway, e.g., phenotypically, genetically, biochemically, and molecularly, manifesting in tuberous sclerosis disease. A defective TSC pathway may be identified as appropriate, e.g. according to a method as conventional.

Tuberous sclerosis include dysfunctions which are neurological in nature and renal dysfunction. Symptoms and diseases associated with tuberous sclerosis disease e.g. include seizures, mental retardation, autism, kidney failure, facial angiofibromas and cardial Rhabdomyomas; cyst-like areas within certain skeletal regions, particularly bones of the fingers and toes (phalanges); characteristic skin lesions including sharply defined areas of decreased skin coloration (hypopigmentation), relatively small reddish nodules that may appear on the cheeks and nose which reddish lesions eventually enlarge, blend together (coalesce), and develop a wart-like appearance (sebaceous adenomas), flat "coffee-colored" areas of increased skin pigmentation (cafe-au-lait spots); benigns, fibrous nodules (fibromas), e.g. arising around or beneath the nails; or rough, elevated, "knobby" lesions (shagreen patches) on the lower back, hypertrophy referring to the enlargement or overgrowth of an organ or body part due to an increase in size of its constituent cells, e.g. including right ventricular hypertrophy, hypertrophic cardiomyopathy, benign prostatic hypertrophy; renal angiomyolipomas (AMLs). e.g. manifesting in heterogeneous, benign tumors, e.g. composed of three distinct cell types including smooth muscle, blood vessel, and adipose cells; a devastating form of lung disease, such as lymphangioleiomyomatosis (LAM), blocked flow of air, blood, and lymph to and from the lungs, kidney tumors associated with LAM, dysapnea, chronic cough, wheezing, pneumothorax, and chest pain, cystic parenchymal destruction, progressive respiratory failure.

By the term "second drug substance" as used herein is meant either any chemotherapeutic agent other than a compound of the present invention, 40-O-(2-hydroxyethyl)-rapamycin. For example, a second drug substance as used herein includes e.g. anticancer drugs, anti-inflammatory and/or immunomodulatory and/or antiallergic drugs, antipruritics, astringent agents, local anesthetics.
For example, in case of a TSC-compound of the present invention, a second drug substance as used herein includes e.g. drugs which are useful in the treatment of symptoms associated with disorders mediated via the Tuberous Sclerosis Complex, such as drugs, useful for the treatment of tuberous sclerosis, renal angiomyolipomas (ALM), lymphangioleiomyomatosis (LAM), subependymal, and/or giant cell astrocytomas (SEGAs) and /or symptoms and/or disorders associated therewith;
Anticancer drugs which are prone to be useful as a combination partner with any compound of the present invention, 40-O-(2-hydroxyethyl)-rapamycin, e.g. prone to be useful according to the present invention, e.g. include
i. a steroid; e.g. prednisone.
ii. an adenosine-kinase-inhibitor; which targets, decreases or inhibits nucleobase, nucleoside, nucleotide and nucleic acid metabolisms, such as 5-lodotubercidin, which is also known as 7H-pyrrolo[2,3-d]pyrimidin-4-amine, 5-iodo-7-β-D-ribofuranosyl-(9Cl).
iii. an adjuvant; which enhances the 5-FU-TS bond as well as a compound which targets, decreases or inhibits, alkaline phosphatase, such as leucovorin, levamisole.
iv. an adrenal cortex antagonist; which targets, decreases or inhibits the activity of the adrenal cortex and changes the peripheral metabolism of corticosteroids, resulting in a decrease in 17-hydroxycorticosteroids, such as mitotane.
v. an AKT pathway inhibitor; such as a compound which targets, decreases or inhibits Akt, also known as protein kinase B (PKB), such as deguelin, which is also known as 3H-bis[1]benzopyrano[3,4-b:6',5'-e]pyran-7(7aH)-one, 13,13a-dihydro-9,10-dimethoxy-3,3-dimethyl-, (7aS, 13aS)-(9Cl); and triciribine, which is also known as 1,4,5,6,8-pentaazaacenaphthylen-3-amine, 1,5-dihydro-5-methyl-1-β-D-ribofuranosyl-(9Cl).
vi. an alkylating agent; which causes alkylation of DNA and results in breaks in the DNA molecules as well as cross-linking of the twin strands, thus interfering with DNA replication and transcription of RNA, such as chlorambucil, chlormethine, cyclophosphamide, ifosfamide, melphalan, estramustine; nitrosueras, such as carmustine, fotemustine, lomustine, streptozocin (streptozotocin, STZ), BCNU; Gliadel; dacarbazine, mechlorethamine, e.g. in the form of a hydrochloride, procarbazine, e.g. in the form of a hydrochloride, thiotepa, temozolomide, nitrogen mustard, mitomycin, altretamine, busulfan, estramustine, uramustine. Cyclophosphamide can be administered, e.g., in the form as it is marketed, e.g., under the trademark CYCLOSTIN®; ifosfamide as HOLOXAN®, temozolomide as TEMODAR®, nitrogen mustard as MUSTARGEN®, estramustine as EMYCT®, streptozocin as ZANOSAR®.
vii. an angiogenesis inhibitor; which targets, decreases or inhibits the production of new blood vessels, e.g. which targets methionine aminopeptidase-2 (MetAP-2), macrophage inflammatory protein-1 (MIP-1alpha), CCL5, TGF-beta, lipoxygenase, cyclooxygenase, and topoisomerase, or which indirectly targets p21, p53, CDK2 and collagen synthesis, e.g. including fumagillin, which is known as 2,4,6,8-decatetraenedioic acid, mono[(3R,4S,5S,6R)-5-methoxy-4-[(2R,3R)-2-methyl-3-(3-methyl-2-butenyl)oxiranyl]-1-oxaspiro[2.5]oct-6-yl] ester, (2E,4E,6E,8E)- (9CI); shikonin, which is also known as 1,4-naphthalenedione, 5,8-dihydroxy-2-[(1R)-1-hydroxy-4-methyl-3-pentenyl]- (9CI); tranilast, which is also known as benzoic acid, 2-[[3-(3,4-dimethoxyphenyl)-1-oxo-2-propenyl]amino]-(9Cl); ursolic acid; suramin; bengamide or a derivative thereof, thalidomide, TNP-470.
viii. an anti-androgen; which blocks the action of androgens of adrenal and testicular origin which stimulate the growth of normal and malignant prostatic tissue, such as nilutamide; bicalutamide (CASODEX®), which can be formulated, e.g., as disclosed in US4636505.
ix. an anti-estrogen; which antagonizes the effect of estrogens at the estrogen receptor level, e.g. including an aromatase inhibitor, which inhibits the estrogen production, i. e. the conversion of the substrates androstenedione and testosterone to estrone and estradiol, respectively, e.g. including atamestane, exemestane, formestane, aminoglutethimide, roglethimide, pyridoglutethimide, trilostane, testolactone, ketokonazole, vorozole, fadrozole, anastrozole, letrozole, toremifene; bicalutamide; flutamide; tamoxifen, tamoxifen citrate; tamoxifen; fulvestrant; raloxifene, raloxifene hydrochloride. Tamoxifen may be e.g. administered in the form as it is marketed, e.g., NOLVADEX®; and raloxifene hydrochloride is marketed as EVISTA®. Fulvestrant may be formulated as disclosed in US4659516 and is marketed as FASLODEX®.
x. an anti-hypercalcemia agent; which is used to treat hypercalcemia, such as gallium (III) nitrate hydrate; and pamidronate disodium.
xi. an antimetabolite; which inhibits or disrupts the synthesis of DNA resulting in cell death, such as 6-mercaptopurine; cytarabine; fludarabine; flexuridine; fluorouracil; 5-fluorouracil(5-FU), floxuridine (5-FUdR), capecitabine; raltitrexed; methotrexate; cladribine; gemcitabine; gemcitabine hydrochloride; thioguanine; 6-thioguanine, hydroxyurea; DNA de-methylating agents, such as 5-azacytidine and decitabine; edatrexate; folic acid antagonists such as pemetrexed. Capecitabine and gemcitabine can be administered e.g. in the marketed form, such as XELODA® and GEMZAR®.
xii. an apoptosis inducer; which induces the normal series of events in a cell that leads to its death, e.g. selectively inducing the X-linked mammalian inhibitor of apoptosis protein XIAP, or e.g. downregulating BCL-xL; such as ethanol, 2-[[3-(2,3-dichlorophenoxy)propyl]amino]-(9Cl); gambogic acid; embelin, which is also known as 2,5-cyclohexadiene-1,4-dione, 2,5-dihydroxy-3-undecyl; arsenic trioxide (TRISENOX®).
xiii. an aurora kinase inhibitor; which targets, decreases or inhibits later stages of the cell cycle from the G2/M check point all the way through to the mitotic checkpoint and late mitosis; such as binucleine 2, which is also known as methanimidamide, N'-[1-(3-chloro-4-fluorophenyl)-4-cyano-1 H-pyrazol-5-yl]-N,N-dimethyl- (9Cl).
xiv. a Bruton's Tyrosine Kinase (BTK) inhibitor; which targets, decreases or inhibits human and murine B cell development; such as terreic acid.
xv. a calcineurin inhibitor; which targets, decreases or inhibits the T cell activation pathway, such as cypermethrin, which is also known as cyclopropanecarboxylic acid, 3-(2,2-dichloroethenyl)-2,2-dimethyl-cyano(3-phenoxyphenyl)methyl ester (9Cl); deltamethrin, which is also known as cyclopropanecarboxylic aci, 3-(2,2-dibromoethenyl)-2,2-dimethyl-(S)-cyano(3-phenoxyphenyl)methyl ester, (1R,3R)-(9Cl); fenvalerate, which is also known as benzeneacetic acid, 4-chloro-α-(1-methylethyl)-cyano(3-phenoxyphenyl)methyl ester (9Cl); and Tyrphostin 8; but excluding cyclosporin or FK506.
xvi. a CaM kinase II inhibitor; which targets, decreases or inhibits CaM kinases; constituting a family of structurally related enzymes that include phosphorylase kinase, myosin light chain kinase, and CaM kinases I-IV; such as 5-isoquinolinesulfonic acid, 4-[(2S)-2-[(5-isoquinolinylsulfonyl)methylamino]-3-oxo-3-(4-phenyl-1-piperazinyl)propyl]phenyl ester (9Cl); benzenesulfonamide, N-[2-[[[3-(4-chlorophenyl)-2-propenyl]methyl]amino]methyl]phenyl]-N-(2-hydroxyethyl)-4-methoxy-(9Cl).
xvii. a CD45 tyrosine phosphatase inhibitor; which targets, decreases or inhibits dephosphorylating regulatory pTyr residues on Src-family protein-tyrosine kinases, which aids in the treatment of a variety of inflammatory and immune disorders; such as phosphonic acid, [[2-(4-bromophenoxy)-5-nitrophenyl]hydroxymethyl]-(9Cl).
xviii. a CDC25 phosphatase inhibitor; which targets, decreases or inhibits overexpressed dephosphorylate cyclin-dependent kinases in tumors; such as 1,4-naphthalenedione, 2,3-bis[(2-hydroyethyl)thio]-(9Cl).
xix. a CHK kinase inhibitor; which targets, decreases or inhibits overexpression of the antiapoptotic protein Bcl-2; such as debromohymenialdisine. Targets of a CHK kinase inhibitor are CHK1 and/or CHK2.
xx. a controlling agent for regulating genistein, olomucine and/or tyrphostins; such as daidzein, which is also known as 4H-1-benzopyran-4-one, 7-hydroxy-3-(4-hydroxyphenyl)-(9Cl); Iso-Olomoucine, and Tyrphostin 1.
xxi. a cyclooxygenase inhibitor; e.g. including Cox-2 inhibitors; which targets, decreases or inhibits the enzyme cox-2 (cyclooxygenase-2); such as 1H-indole-3-acetamide, 1-(4-chlorobenzoyl)-5-methoxy-2-methyl-N-(2-phenylethyl)-(9Cl); 5-alkyl substituted 2-arylaminophenylacetic acid and derivatives, e.g. celecoxib (CELEBREX®), rofecoxib (VIOXX®), etoricoxib, valdecoxib; or a 5-alkyl-2-arylaminophenylacetic acid, e.g., 5-methyl-2-(2'-chloro-6'-fluoroanilino)phenyl acetic acid, lumiracoxib; and celecoxib.
xxii. a cRAF kinase inhibitor; which targets, decreases or inhibits the up-regulation of E-selectin and vascular adhesion molecule-1 induced by TNF; such as 3-(3,5-dibromo-4-hydroxybenzylidene)-5-iodo-1,3-dihydroindol-2-one; and benzamide, 3-(dimethylamino)-N-[3-[(4-hydroxybenzoyl)amino]-4-methylphenyl]-(9Cl). Raf kinases play an important role as extracellular signal-regulating kinases in cell differentiation, proliferation, and apoptosis. A target of a cRAF kinase inhibitor includes, but is not limited, to RAF1.
xxiii. a cyclin dependent kinase inhibitor; which targets, decreases or inhibits cyclin dependent kinase playing a role in the regulation of the mammalian cell cycle; such as N9-isopropyl-olomoucine; olomoucine; purvalanol B, which is also known as Benzoic acid, 2-chloro-4-[[2-[[(1R)-1-(hydroxymethyl)-2-methylpropyl]amino]-9-(1-methylethyl)-9H-purin-6-yl]amino]- (9CI); roascovitine; indirubin, which is also known as 2H-indol-2-one, 3-(1,3-dihydro-3-oxo-2H-indol-2-ylidene)-1,3-dihydro- (9CI); kenpaullone, which is also known as indolo[3,2-d][1]benzazepin-6(5H)-one, 9-bromo-7,12-dihydro- (9CI); purvalanol A, which is also known as 1-Butanol, 2-[[6-[(3-chlorophenyl)amino]-9-(1-methylethyl)-9H-purin-2-yl]amino]-3-methyl-, (2R)- (9CI); indirubin-3'-monooxime. Cell cycle progression is regulated by a series of sequential events that include the activation and subsequent inactivation of cyclin dependent kinases (Cdks) and cyclins. Cdks are a group of serine/threonine kinases that form active heterodimeric complexes by binding to their regulatory subunits, cyclins. Examples of targets of a cyclin dependent kinase inhibitor include, but are not limited to, CDK, AHR, CDK1, CDK2, CDK5, CDK4/6, GSK3beta, and ERK.
xxiv. a cysteine protease inhibitor; which targets, decreases or inhibits cystein protease which plays a vital role in mammalian cellular turnover and apotosis; such as 4-morpholinecarboxamide,N-[(1S)-3-fluoro-2-oxo-1-(2-phenylethyl)propyl]amino]-2-oxo-1-(phenylmethyl)ethyl]-(9Cl).
xxv. a DNA intercalator; which binds to DNA and inhibits DNA, RNA, and protein synthesis; such as plicamycin, dactinomycin.
xxvi. a DNA strand breaker; which causes DNA strand scission and results in inhibition of DNA synthesis, inhibition of RNA and protein synthesis; such as bleomycin.
xxvii. an E3 Ligase inhibitor; which targets, decreases or inhibits the E3 ligase which inhibits the transfer of ubiquitin chains to proteins, marking them for degradation in the proteasome; such as N-((3,3,3-trifluoro-2-trifluoromethyl)propionyl)sulfanilamide.
xxviii. an endocrine hormone; which by acting mainly on the pituitary gland causes the suppression of hormones in males, the net effect being a reduction of testosterone to castration levels; in females, both ovarian estrogen and androgen synthesis being inhibited; such as leuprolide; megestrol, megestrol acetate.
xxix. compounds targeting, decreasing or inhibiting the activity of the epidermal growth factor family of receptor tyrosine kinases (EGFR, ErbB2, ErbB3, ErbB4 as homo- or heterodimers), such as compounds, proteins or antibodies which inhibit members of the EGF receptor tyrosine kinase family, e.g. EGF receptor, ErbB2, ErbB3 and ErbB4 or bind to EGF or EGF-related ligands, and are in particular those compounds, proteins or monoclonal antibodies generically and specifically disclosed in WO 9702266, e.g. the compound of ex. 39, EP0564409, WO9903854, EP0520722, EP0566226, EP0787722, EP0837063, US5747498, WO9810767, WO9730034, WO9749688, WO9738983 and, especially, WO9630347, e.g. a compound known as CP 358774, WO9633980, e.g. a compound known as ZD 1839; and WO 9503283, e.g. a compound known as ZM105180, e.g including trastuzumab (HERCEPTIN®), cetuximab, iressa, OSI-774, CI-1033, EKB-569, GW-2016, E1.1, E2.4, E2.5, E6.2, E6.4, E2.11, E6.3 or E7.6.3, 7H-pyrrolo-[2,3-d]pyrimidine derivatives which are e.g. disclosed in WO03013541, erlotinib, gefitinib. Erlotinib can be administered in the form as it is marketed, e.g. TARCEVA®, and gefitinib as IRESSA®, human monoclonal antibodies against the epidermal growth factor receptor including ABX-EGFR.
xxx. an EGFR, PDGFR tyrosine kinase inhibitor; such as EGFR kinase inhibitors including tyrphostin 23, tyrphostin 25, tyrphostin 47, tyrphostin 51 and tyrphostin AG 825; 2-propenamide, 2-cyano-3-(3,4-dihydroxyphenyl)-N-phenyl-(2E)-(9Cl); tyrphostin Ag 1478; lavendustin A; 3-pyridineacetonitrile, α-[(3,5-dichlorophenyl)methylene]-, (αZ)-(9CI); an example of an EGFR, PDGFR tyrosine kinase inhibitor e.g. includes tyrphostin 46. PDGFR tyrosine kinase inhibitor including tyrphostin 46. Targets of an EGFR kinase inhibitor include guanylyl cyclase (GC-C) HER2, EGFR, PTK and tubulin.
xxxi. a farnesyltransferase inhibitor; which targets, decreases or inhibits the Ras protein; such as α-hydroxyfarnesylphosphonic acid; butanoic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-,1-methylethyl ester, (2S)-(9cl); manumycin A; L-744,832 or DK8G557, tipifarnib (R115777), SCH66336 (Ionafarnib), BMS-214662,
xxxii. a Flk-1 kinase inhibitor; which targets, decreases or inhibits Flk-1 tyrosine kinase activity; such as 2-propenamide, 2-cyano-3-[4-hydroxy-3,5-bis(1-methylethyl)phenyl]-N-(3-phenylpropyl)-(2E)-(9Cl). A target of a Flk-1 kinase inhibitor includes, but is not limited to, KDR.
xxxiii. a Glycogen synthase kinase-3 (GSK3) inhibitor; which targets, decreases or inhibits glycogen synthase kinase-3 (GSK3); such as indirubin-3'-monooxime. Glycogen Synthase Kinase-3 (GSK-3; tau protein kinase I), a highly conserved, ubiquitously expressed serine/threonine protein kinase, is involved in the signal transduction cascades of multiple cellular processes. which is a protein kinase that has been shown to be involved in the regulation of a diverse array of cellular functions, including protein synthesis, cell proliferation, cell differentiation, microtubule assembly/disassembly, and apoptosis.
xxxiv. a histone deacetylase (HDAC) inhibitor; which inhibits the histone deacetylase and which possess anti-proliferative activity; such as compounds disclosed in WO0222577, especially N-hydroxy-3-[4-[[(2-hydroxyethyl)[2-(1H-indol-3-yl)ethyl]-amino]methyl]phenyl]-2E-2-propenamide, and N-hydroxy-3-[4-[[[2-(2-methyl-1H-indol-3-yl)-ethyl]-amino]methyl]phenyl]-2E-2-propenamide and pharmaceutically acceptable salts thereof; suberoylanilide hydroxamic acid (SAHA); [4-(2-amino-phenylcarbamoyl)-benzyl]-carbamic acid pyridine-3-ylmethyl ester and derivatives thereof; butyric acid, pyroxamide, trichostatin A, oxamflatin, apicidin, depsipeptide; depudecin; trapoxin, HC toxin, which is also known as cyclo[L-alanyl-D-alanyl-(S,2S)- -amino- - oxooxiraneoctanoyl-D-prolyl] (9Cl); sodium phenylbutyrate, suberoyl bis-hydroxamic acid; Trichostatin A, BMS-27275, pyroxamide, FR-901228, valproic acid.
xxxv. a HSP90 inhibitor; which targets, decreases or inhibits the intrinsic ATPase activity of HSP90; degrades, targets, decreases or inhibits the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin derivative; other geldanamycin-related compounds; radicicol and HDAC inhibitors. Other examples of an HSP90 inhibitor include geldanamycin, 17-demethoxy-17-(2-propenylamino)-(9Cl). Potential indirect targets of an HSP90 inhibitor include FLT3, BCR-ABL, CHK1, CYP3A5*3 and/or NQ01*2.
xxxvi.a I-kappa B-alpha kinase inhibitor (IKK); which targets, decreases or inhibits NF-kappaB, such as 2-propenenitrile, 3-[(4-methylphenyl)sulfonyl]-(2E)-(9Cl).
xxxvii. an insulin receptor tyrosine kinase inhibitor; which modulates the activities of phosphatidylinositol 3-kinase, microtubule-associated protein, and S6 kinases; such as hydroxyl-2-naphthalenylmethylphosphonic acid, LY294002.
xxxviii.a c-Jun N-terminal kinase (JNK) kinase inhibitor; which targets, decreases or inhibits Jun N-terminal kinase; such as pyrazoleanthrone and/or epigallocatechin gallate. Jun N-terminal kinase (JNK), a serine-directed protein kinase, is involved in the phosphorylation and activation of c-Jun and ATF2 and plays a significant role in metabolism, growth, cell differentiation, and apoptosis. A target for a JNK kinase inhibitor includes, but is not limited to, DNMT.
xxxix a microtubule binding agent; which acts by disrupting the microtubular network that is essential for mitotic and interphase cellular function; such as vinblastine, vinblastine sulfate; vinca alkaloids, such as vincristine, vincristine sulfate; vindesine; vinorelbine; taxanes, such as docetaxel; paclitaxel; discodermolides; colchicine, epothilones and derivatives thereof, e.g. epothilone B or a derivative thereof. Paclitaxel is marketed as TAXOL®; docetaxel as TAXOTERE®; vinblastine sulfate as VINBLASTIN R.P®; and vincristine sulfate as FARMISTIN®. Also included are the generic forms of paclitaxel as well as various dosage forms of paclitaxel. Generic forms of paclitaxel include, but are not limited to, betaxolol hydrochloride. Various dosage forms of paclitaxel include, but are not limited to albumin nanoparticle paclitaxel marketed as ABRAXANE®; ONXOL®, CYTOTAX®. Discodermolide can be obtained, e.g., as disclosed in US5010099. Also included are Epotholine derivatives which are disclosed in US6194181, WO98/0121, WO9825929, WO9808849, WO9943653, WO9822461 and WO0031247. Especially preferred are Epotholine A and/or B.
xl. a mitogen-activated protein (MAP) kinase-inhibitor; which targets, decreases or inhibits Mitogen-activated protein, such as benzenesulfonamide, N-[2-[[[3-(4-chlorophenyl)-2-propenyl]methyl]amino]methyl]phenyl]-N-(2-hydroxyethyl)-4-methoxy-(9Cl). The mitogen-activated protein (MAP) kinases are a group of protein serine/threonine kinases that are activated in response to a variety of extracellular stimuli and mediate signal transduction from the cell surface to the nucleus. They regulate several physiological and pathological cellular phenomena, including inflammation, apoptotic cell death, oncogenic transformation, tumor cell invasion, and metastasis.
xli. a MDM2 inhibitor; which targets, decreases or inhibits the interaction of MDM2 and the p53 tumor suppressor; such as trans-4-iodo, 4'-boranyl-chalcone.
xlii. a MEK inhibitor; which targets, decreases or inhibits the kinase activity of MAP kinase MEK; such as Nexavar® (sorafenib tosylate), butanedinitrile, bis[amino[2-aminophenyl)thio]methylene]-(9Cl). A target of a MEK inhibitor includes, but is not limited to ERK. An indirect target of a MEK inhibitor includes, but is not limited to, cyclin D1.
xliii: a matrix metalloproteinase inhibitor (MMP) inhibitor; which targets, decreases or inhibits a class of protease enzyme that selectively catalyze the hydrolysis of polypeptide bonds including the enzymes MMP-2 and MMP-9 that are involved in promoting the loss of tissue structure around tumors and facilitating tumor growth, angiogenesis, and metastasis such as actinonin, which is also known as butanediamide, N-4-hydroxy-N1-[(1S)-1-[[(2S)-2-(hydroxymethyl)-1-pyrrolidinyl]carbonyl]-2-methylpropyl]-2-pentyl-, (2R)-(9Cl); epigallocatechin gallate; collagen peptidomimetic and non-peptidomimetic inhibitors; tetracycline derivatives, e.g., hydroxamate peptidomimetic inhibitor batimastat; and its orally-bioavailable analogue marimastat, prinomastat, metastat, neovastat, tanomastat, TAA211, BMS-279251, BAY 12-9566, MMI270B or AAJ996. A target of a MMP inhibitor includes, but is not limited to, polypeptide deformylase.
xliv. a NGFR tyrosine-kinase-inhibitor; which targets, decreases or inhibits nerve growth factor dependent p140^{c-*trk*} tyrosine phosphorylation; such as tyrphostin AG 879. Targets of a NGFR tyrosine-kinase-inhibitor include, but are not limited to, HER2, FLK1, FAK, TrkA, and/or TrkC. An indirect target inhibits expression of RAF1.
xlv. a p38 MAP kinase inhibitor, including a SAPK2/p38 kinase inhibitor; which targets, decreases or inhibits p38-MAPK, which is a MAPK family member, such as phenol, 4-[4-(4-fluorophenyl)-5-(4-pyridinyl)-1H-imidazol-2-yl]-(9Cl). An example of a a SAPK2/p38 kinase inhibitor includes, but is not limited to, benzamide, 3-(dimethylamino)-N-[3-[(4-hydroxybenzoyl)amino]-4-methylphenyl]-(9Cl). A MAPK family member is a serine/threonine kinase activated by phosphorylation of tyrosine and threonine residues. This kinase is phosphorylated and activated by many cellular stresses and inflammatory stimuli, thought to be involved in the regulation of important cellular responses such as apoptosis and inflammatory reactions.
xlvi. a p56 tyrosine kinase inhibitor; which targets, decreases or inhibits p56 tyrosine kinase, which is an enzyme that is a lymphoid-specific src family tyrosine kinase critical for T-cell development and activation; such as damnacanthal, which is also known as 2-anthracenecarboxaldehyde-9,10-dihydro-3-hydroxy-1methoxy-9,10-dioxo-(9Cl), Tyrphostin 46. A target of a p56 tyrosine kinase inhibitor includes, but is not limited to, Lck. Lck is associated with the cytoplasmic domains of CD4, CD8 and the beta-chain of the IL-2 receptor, and is thought to be involved in the earliest steps of TCR-mediated T-cell activation.
xlvii. a PDGFR tyrosine kinase inhibitor; targeting, decreasing or inhibiting the activity of the C-kit receptor tyrosine kinases (part of the PDGFR family), such as targeting, decreasing or inhibiting the activity of the c-Kit receptor tyrosine kinase family, especially inhibiting the c-Kit receptor. Examples of targets of a PDGFR tyrosine kinase inhibitor includes, but are not limited to PDGFR, FLT3 and/or c-KIT; such as tyrphostin AG 1296; tyrphostin 9; 1,3-butadiene-1,1,3-tricarbonitrile,2-amino-4-(1H-indol-5-yl)-(9Cl); N-phenyl-2-pyrimidine-amine derivative, e. g. imatinib, IRESSA®. PDGF plays a central role in regulating cell proliferation, chemotaxis, and survival in normal cells as well as in various disease states such as cancer, atherosclerosis, and fibrotic disease. The PDGF family is composed of dimeric isoforms (PDGF-AA, PDGF-BB, PDGF-AB, PDGF-CC, and PDGF-DD), which exert their cellular effects by differentially binding to two receptor tyrosine kinases. PDGFR-*α* and PDGFR-β have molecular masses of ∼ 170 and 180 kDa, respectively.
xlviii. a phosphatidylinositol 3-kinase inhibitor; which targets, decreases or inhibits PI 3-kinase; such as wortmannin, which is also known as 3H-Furo[4,3,2-de]indeno[4,5-h]-2-benzopyran-3,6,9-trione, 11-(acetyloxy)-1,6b,7,8,9a, 10,11,11b-octahydro-1-(methoxymethyl)-9a,11b-dimethyl-, (1S,6bR,9aS,11R,11bR)- (9CI); 8-phenyl-2-(morpholin-4-yl)-chromen-4-one; quercetin, quercetin dihydrate. PI 3-kinase activity has been shown to increase in response to a number of hormonal and growth factor stimuli, including insulin, platelet-derived growth factor, insulin-like growth factor, epidermal growth factor, colony-stimulating factor, and hepatocyte growth factor, and has been implicated in processes related to cellular growth and transformation. An example of a target of a phosphatidylinositol 3-kinase inhibitor includes, but is not limited to, Pi3K.
xlix. a phosphatase inhibitor; which targets, decreases or inhibits phosphatase; such as cantharidic acid; cantharidin; and L-leucinamide, N-[4-(2-carboxyethenyl)benzoyl]glycyl-L-α-glutamyl-(E)-(9Cl). Phosphatases remove the phosphoryl group and restore the protein to its original dephosphorylated state. Hence, the phosphorylation- dephosphorylation cycle can be regarded as a molecular "on-off" switch.
l. a platinum agent; which contains platinum and inhibit DNA synthesis by forming interstrand and intrastrand cross-linking of DNA molecules; such as carboplatin; cisplatin; oxaliplatin; cisplatinum; satraplatin and platinum agents such as ZD0473. Carboplatin can be administered, e.g., in the form as it is marketed, e.g. CARBOPLAT®; and oxaliplatin as ELOXATIN®.
li. a protein phosphatase inhibitor, including a PP1 and PP2 inhibitor and a tyrosine phosphatase inhibitor; which targets, decreases or inhibits protein phosphatase. Examples of a PP1 and PP2A inhibitor include cantharidic acid and/or cantharidin. Examples of a tyrosine phosphatase inhibitor include, but are not limited to, L-P-bromotetramisole oxalate; 2(5H)-furanone,4-hydroxy-5-(hydroxymethyl)-3-(1-oxohexadecyl)-, (5R)-(9Cl); and benzylphosphonic acid.
   The term "a PP1 or PP2 inhibitor", as used herein, relates to a compound which targets, decreases or inhibits Ser/Thr protein phosphatases. Type I phosphatases, which include PP1, can be inhibited by two heat-stable proteins known as Inhibitor-1 (I-1) and Inhibitor-2 (I-2). They preferentially dephosphorylate a subunit of phosphorylase kinase. Type II phosphatases are subdivided into spontaneously active (PP2A), CA²⁺-dependent (PP2B), and Mg²⁺-dependent (PP2C) classes of phosphatases.
   The term "tyrosine phosphatase inhibitor", as used here, relates to a compounds which targets, decreases or inhibits tyrosine phosphatase. Protein tyrosine phosphatases (PTPs) are relatively recent additions to the phosphatase family. They remove phosphate groups from phosphorylated tyrosine residues of proteins. PTPs display diverse structural features and play important roles in the regulation of cell proliferation, differentiation, cell adhesion and motility, and cytoskeletal function. Examples of targets of a tyrosine phosphatase inhibitor include, but are not limited to, alkaline phosphatase (ALP), heparanase, PTPase, and/or prostatic acid phosphatase.
lii. a PKC inhibitor and a PKC delta kinase inhibitor: The term "a PKC inhibitor", as used herein, relates to a compound which targets, decreases or inhibits protein kinase C as well as its isozymes. Protein kinase C (PKC), a ubiquitous, phospholipid-dependent enzyme, is involved in signal transduction associated with cell proliferation, differentiation, and apoptosis. Examples of a target of a PKC inhibitor include, but are not limited to, MAPK and/or NF-kappaB. Examples of a PKC inhibitor include, but are not limited to, 1-H-pyrrolo-2,5-dione,3-[1-[3-(dimethylamino)propyl]-1H-indol-3-yl]-4-(1H-indol-3-yl)-(9Cl); bisindolylmaleimide IX; sphingosine, which is known as 4-octadecene-1,3-diol, 2-amino-, (2S,3R,4E)- (9CI); staurosporine, which is known as 9,13-Epoxy-1H,9H-diindolo[1,2,3-gh:3',2',1'-lm]pyrrolo[3,4-j][1,7]benzodiazonin-1-one, staurosporine derivatives such as disclosed in EP0296110, e. g. midostaurin; 2,3,10,11,12,13-hexahydro-10-methoxy-9-methyl-11-(methylamino)-, (9S,10R,11R,13R)- (9CI); tyrphostin 51; and hypericin, which is also known as phenanthro[1,10,9,8-opqra]perylene-7,14-dione, 1,3,4,6,8,13-hexahydroxy-10,11-dimethyl-, stereoisomer (6CI,7CI,8CI,9CI), UCN-01,safingol, BAY 43-9006, bryostatin 1, perifosine;llmofosine ; RO 318220 and RO 320432; GO 6976 ; Isis 3521; LY333531/LY379196. The term "a PKC delta kinase inhibitor", as used herein, relates to a compound which targets, decreases or inhibits the delta isozymes of PKC. The delta isozyme is a conventional PKC isozymes and is Ca²⁺-dependent. An example of a PKC delta kinase inhibitor includes, but is not limited to, Rottlerin, which is also known as 2-Propen-1-one, 1-[6-[(3-acetyl-2,4,6-trihydroxy-5-methylphenyl)methyl]-5,7-dihydroxy-2,2-dimethyl-2H-1-benzopyran-8-yl]-3-phenyl-, (2E)- (9Cl).
liii. a polyamine synthesis inhibitor; which targets, decreases or inhibits polyamines spermidine; such as DMFO, which is also known as (-)-2-difluoromethylornithin; N1, N12-diethylspermine 4HCl. The polyamines spermidine and spermine are of vital importance for cell proliferation, although their precise mechanism of action is unclear. Tumor cells have an altered polyamine homeostasis reflected by increased activity of biosynthetic enzymes and elevated polyamine pools.
liv. a proteosome inhibitor; which targets, decreases or inhibits proteasome, such as aclacinomycin A; gliotoxin; PS-341; MLN 341; bortezomib; velcade. Examples of targets of a proteosome inhibitor include, but are not limited to, O(2)(-)-generating NADPH oxidase, NF-kappaB, and/or farnesyltransferase, geranyltransferase I.
lv. a PTP1 B inhibitor; which targets, decreases or inhibits PTP1 B, a protein tyrosine kinase inhibitor; such as L-leucinamide, N-[4-(2-carboxyethenyl)benzoyl]glycyl-L-α-glutamyl-,(E)-(9Cl).
lvi. a protein tyrosine kinase inhibitor including a SRC family tyrosine kinase inhibitor; a Syk tyrosine kinase inhibitor; and a JAK-2 and/or JAK-3 tyrosine kinase inhibitor; The term "a protein tyrosine kinase inhibitor", as used herein, relates to a compound which targets, decreases or inhibits protein tyrosine kinases. Protein tyrosine kinases (PTKs) play a key role in the regulation of cell proliferation, differentiation, metabolism, migration, and survival. They are classified as receptor PTKs and non-receptor PTKs. Receptor PTKs contain a single polypeptide chain with a transmembrane segment. The extracellular end of this segment contains a high affinity ligand-binding domain, while the cytoplasmic end comprises the catalytic core and the regulatory sequences. Examples of targets of a tyrosine kinase inhibitor include, but are not limited to, ERK1, ERK2, Bruton's tyrosine kinase (Btk), JAK2, ERK½, PDGFR, and/or FLT3. Examples of indirect targets include, but are not limited to, TNFalpha, NO, PGE2, IRAK, iNOS, ICAM-1, and/or E-selectin. Examples of a tyrosine kinase inhibitor include, but are not limited to, tyrphostin AG 126; tyrphostin Ag 1288; tyrphostin Ag 1295; geldanamycin; and genistein.
   Non-receptor tyrosine kinases include members of the Src, Tec, JAK, Fes, Abl, FAK, Csk, and Syk families. They are located in the cytoplasm as well as in the nucleus. They exhibit distinct kinase regulation, substrate phosphorylation, and function. Deregulation of these kinases has also been linked to several human diseases.
   The term "a SRC family tyrosine kinase inhibitor", as used herein, relates to a compound which targets, decreases or inhibits SRC. Examples of a SRC family tyrosine kinase inhibitor include, but are not limited to, PP1, which is also known as 1 H-pyrazolo[3,4-d]pyrimidin-4-amine, 1-(1,1-dimethylethyl)-3-(1-naphthalenyl)- (9CI); and PP2, which is also known as 1H-Pyrazolo[3,4-d]pyrimidin-4-amine, 3-(4-chlorophenyl)-1-(1,1-dimethylethyl)- (9Cl).
   The term "a Syk tyrosine kinase inhibitor", as used herein, relates to a compound which targets, decreases or inhibits Syk. Examples of targets for a Syk tyrosine kinase inhibitor include, but are not limited to, Syk, STAT3, and/or STAT5. An example of a Syk tyrosine kinase inhibitor includes, but is not limited to, piceatannol, which is also known as 1,2-benzenediol, 4-[(1E)-2-(3,5-dihydroxyphenyl)ethenyl]- (9Cl).
   The term "a Janus (JAK-2 and/or JAK-3) tyrosine kinase inhibitor", as used herein, relates to a compound which targets, decreases or inhibits janus tyrosine kinase. Janus tyrosine kinase inhibitor are shown anti-leukemic agents with anti-thrombotic, anti-allergic and immunosuppressive properties. Targets of a JAK-2 and/or JAK-3 tyrosine kinase inhibitor include, but are not limited to, JAK2, JAK3, STAT3. An indirect target of an JAK-2 and/or JAK-3 tyrosine kinase inhibitor includes, but is not limited to CDK2. Examples of a JAK-2 and/or JAK-3 tyrosine kinase inhibitor include, but are not limited to, Tyrphostin AG 490; and 2-naphthyl vinyl ketone.
   Compounds which target, decrease or inhibit the activity of c-Abl family members and their gene fusion products, e. g. include PD180970 ; AG957; or NSC 680410.
lvii. a retinoid; which target, decrease or inhibit retinoid dependent receptors; such as isotretinoin, tretinoin.
lviii. a RNA polymerase II elongation inhibitor; which targets, decreases or inhibits insulin-stimulated nuclear and cytosolic p70S6 kinase in CHO cells; targets, decreases or inhibits RNA polymerase II transcription, which may be dependent on casein kinase II; and targets, decreases or inhibits germinal vesicle breakdown in bovine oocytes; such as 5,6-dichloro-1-beta-D-ribofuranosylbenzimidazole.
lvix. a serine/threonine kinase inhibitor; which inhibits serine/threonine kinases; such as 2-aminopurine, also known as 1H-purin-2-amine(9Cl). An example of a target of a serine/threonine kinase inhibitor includes, but is not limited to, dsRNA-dependent protein kinase (PKR). Examples of indirect targets of a serine/threonine kinase inhibitor include, but are not limited to, MCP-1, NF-kappaB, elF2alpha, COX2, RANTES, IL8,CYP2A5, IGF-1, CYP2B1, CYP2B2, CYP2H1, ALAS-1, HIF-1, erythropoietin, and/or CYP1A1.
lx. a sterol biosynthesis inhibitor; which inhibits the biosynthesis of sterols such as cholesterol; such as terbinadine. Examples of targets for a sterol biosynthesis inhibitor include, but are not limited to, squalene epoxidase, and CYP2D6.
lxi. a topoisomerase inhibitor; including a topoisomerase I inhibitor and a topoisomerase II inhibitor. Examples of a topoisomerase I inhibitor include, but are not limited to, topotecan, gimatecan, irinotecan, camptothecan and its analogues, 9-nitrocamptothecin and the macromolecular camptothecin conjugate PNU-166148 (the compound designated as A1 in WO9917804); 10-hydroxycamptothecin acetate salt; etoposide; idarubicin hydrochloride; irinotecan hydrochloride; teniposide; topotecan, topotecan hydrochloride; doxorubicin; epirubicin, epirubicin hydrochloride; mitoxantrone, mitoxantrone hydrochloride; daunorubicin, daunorubicin hydrochloride, dasatinib (BMS-354825). Irinotecan can be administered, e.g., in the form as it is marketed, e.g., under the trademark CAMPTOSAR®. Topotecan can be administered, e.g., in the form as it is marketed, e.g., under the trademark HYCAMTIN®. The term "topoisomerase II inhibitor", as used herein, includes, but is not limited to, the anthracyclines, such as doxorubicin, including liposomal formulation, e.g., CAELYX®, daunorubicin, including liposomal formulation, e.g., DAUNOSOME®, epirubicin, idarubicin and nemorubicin; the anthraquinones mitoxantrone and losoxantrone; and the podophillotoxines etoposide and teniposide. Etoposide is marketed as ETOPOPHOS®; teniposide as VM 26-BRISTOL®; doxorubicin as ADRIBLASTIN® or ADRIAMYCIN®; epirubicin as FARMORUBICIN® idarubicin as ZAVEDOS®; and mitoxantrone as NOVANTRON®.
lxii. VEGFR tyrosine kinase inhibitor; which targets, decreases and/or inhibits the known angiogenic growth factors and cytokines implicated in the modulation of normal and pathological angiogenesis. The VEGF family (VEGF-A, VEGF-B, VEGF-C, VEGF-D) and their corresponding receptor tyrosine kinases [VEGFR-1 (Flt-1), VEGFR-2 (Flk-1, KDR), and VEGFR-3 (Flt-4)] play a paramount and indispensable role in regulating the multiple facets of the angiogenic and lymphangiogenic processes. An example of a VEGFR tyrosine kinase inhibitor includes 3-(4-dimethylaminobenzylidenyl)-2-indolinone. Compounds which target, decrease or inhibit the activity of VEGFR are especially compounds, proteins or antibodies which inhibit the VEGF receptor tyrosine kinase, inhibit a VEGF receptor or bind to VEGF, and are in particular those compounds, proteins or monoclonal antibodies generically and specifically disclosed in WO9835958, e. g.1- (4- chloroanilino)-4- (4-pyridylmethyl) phthalazine or a pharmaceutical acceptable salt thereof, e. g. the succinate, or in WO0009495, WO0027820, WO0059509, WO9811223, WO0027819 and EP0769947; e.g. those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58,1998,3209-3214, and by J. Mordenti et al in Toxicologic Pathology, Vol. 27, no. 1, pp 14-21,1999; in WO0037502 and WO9410202; Angiostatin, described by M. S. O'Reilly et al, Cell 79,1994,315-328; Endostatin described by M. S. O'Reilly et al, Cell 88,1997,277-285;anthranilic acid amides; ZD4190; ZD6474; SU5416; SU6668; or anti-VEGF antibodies or anti-VEGF receptor antibodies, e. g. RhuMab (bevacizumab). By antibody is meant intact monoclonal antibodies, polyclonal antibodies, multispecific antibodies formed from at least 2 intact antibodies, and antibodies fragments so long as they exhibit the desired biological activity. An example of an VEGF-R2 inhibitor e.g. includes axitinib,
lxiii. a gonadorelin agonist, such as abarelix, goserelin, goserelin acetate,
lxiv. a compound which induce cell differentiation processes, such as retinoic acid, alpha-, gamma- or 8- tocopherol or alpha-, gamma- or 8-tocotrienol.
lxv. a bisphosphonate, e.g. including etridonic, clodronic, tiludronic, pamidronic, alendronic, ibandronic, risedronic and zoledronic acid.
lxvi. a heparanase inhibitor which prevents heparan sulphate degradation, e. g. PI-88,
lxvii. a biological response modifier, preferably alymphokine or interferons, e. g. interferon alpha,
lxviii. a telomerase inhibitor, e. g. telomestatin,
lxix. mediators, such as inhibitors of catechol-O-methyltransferase, e.g. entacapone,
lxx: ispinesib, permetrexed (Alimta®), sunitinib (SU11248), diethylstilbestrol (DES), BMS224818 (LEA29Y),
lxxi somatostatin or a somatostatin analogue, such as octreotide (Sandostatin® or Sandostatin LAR®).
lxxii. Growth Hormone-Receptor Antagonists, such as pegvisomant, filgrastim or pegfilgrastim, or interferon alpha.

Treatment in combination with an anticancer drug, such as indicated herein, may be associated with radiotherapy.

Anti-inflammatory and/or immunomodulatory drugs which are prone to be useful in combination with a compound of the present invention, 40-O-(2-hydroxyethyl)-rapamycin, e.g. prone to be useful according to the present invention, e.g. include
- mediators, e.g. inhibitors, of calcineurin, e.g. cyclosporin A, FK 506;
- ascomycins having immuno-suppressive properties, e.g. ABT-281, ASM981;
- corticosteroids; cyclophosphamide; azathioprene; leflunomide; mizoribine;
- mycophenolic acid or salt; e.g. sodium, mycophenolate mofetil;
- 15-deoxyspergualine or an immunosuppressive homologue, analogue or derivative thereof;
- mediators, e.g. inhibitors, of bcr-abl tyrosine kinase activity;
- mediators, e.g. inhibitors, of c-kit receptor tyrosine kinase activity;
- mediators, e.g. inhibitors, of PDGF receptor tyrosine kinase activity, e.g. Gleevec (imatinib);
- mediators, e.g. inhibitors, of p38 MAP kinase activity,
- mediators, e.g. inhibitors, of VEGF receptor tyrosine kinase activity,
- mediators, e.g. inhibitors, of PKC activity, e.g. as disclosed in WO0238561 or WO0382859, e.g. the compound of Example 56 or 70;
- mediators, e.g. inhibitors, of JAK3 kinase activity, e.g. N-benzyl-3,4-dihydroxy-benzylidene-cyanoacetamide α-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550), or a compound as disclosed in WO2004052359 or WO2005066156;
- mediators, e.g. agonists or modulators of S1 P receptor activity, e.g. FTY720 optionally phosphorylated or an analog thereof, e.g. 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-benzyl}-azetidine-3-carboxylic acid or its pharmaceutically acceptable salts;
- immunosuppressive monoclonal antibodies, e.g., monoclonal antibodies to leukocyte receptors, e.g., Blys/BAFF receptor, MHC, CD2, CD3, CD4, CD7, CD8, CD25, CD28, CD40, CD45, CD52, CD58, CD80, CD86, IL-12 receptor, IL-17 receptor, IL-23 receptor or their ligands;
- other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4lg (for ex. designated ATCC 68629) or a mutant thereof, e.g. LEA29Y;
- mediators, e.g. inhibitors of adhesion molecule activities, e.g. LFA-1 antagonists, ICAM-1 or -3 antagonists, VCAM-4 antagonists or VLA-4 antagonists,
- mediators, e.g. antagonists of CCR9 acitiviy,
- mediators, e.g. inhibitors, of MIF activity,
- 5-aminosalicylate (5-ASA) agents, such as sulfasalazine, Azulfidine®, Asacol®, Dipentum®, Pentasa®, Rowasa®, Canasa®, Colazal®, e.g. drugs containing mesalamine; e.g mesalazine in combination with heparin;
- mediators, e.g. inhibitors, of TNF-alpha activity, e.g. including antibodies which bind to TNF-alpha, e.g. infliximab (Remicade®), thalidomide, lenalidomide,
- nitric oxide releasing non-steriodal anti-inlammatory drugs (NSAIDs), e.g. including COX-inhibiting NO-donating drugs (CINOD);
- phospordiesterase, e.g. mediators, such as inhibitors of PDE4B activity,
- mediators, e.g. inhibitors, of caspase activity,
- mediators, e.g. agonists, of the G protein coupled receptor GPBAR1,
- mediators, e.g. inhibitors, of ceramide kinase activity,
- 'multi-functional anti-inflammatory' drugs (MFAIDs), e.g. cytosolic phoshpholipase A2 (cPLA2) inhibitors, such as membrane-anchored phospholipase A2 inhibitors linked to glycosaminoglycans;
- antibiotics, such as penicillins, cephalosporins, erythromycins, tetracyclines, sulfonamides, such as sulfadiazine, sulfisoxazole; sulfones, such as dapsone; pleuromutilins, fluoroquinolones, e.g. metronidazole, quinolones such as ciprofloxacin; levofloxacin; probiotics and commensal bacteria e.g. Lactobacillus, Lactobacillus reuteri;
- antiviral drugs, such as ribivirin, vidarabine, acyclovir, ganciclovir, zanamivir, oseltamivir phosphate, famciclovir, atazanavir, amantadine, didanosine, efavirenz, foscarnet, indinavir, lamivudine, nelfinavir, ritonavir, saquinavir, stavudine, valacyclovir, valganciclovir, zidovudine.

Anti-inflammatory drugs which are prone to be useful in combination with a compound of the present invention, 40-O-(2-hydroxyethyl)-rapamycin , e.g. prone to be useful according to the present invention, include e.g. non-steroidal antiinflammatory agents (NSAIDs) such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone); cyclooxygenase-2 (COX- 2) inhibitors such as celecoxib; inhibitors of phosphodiesterase type IV (PDE-IV); antagonists of the chemokine receptors, especially CCR-1, CCR-2, and CCR-3; cholesterol lowering agents such as HMG-CoA reductase inhibitors (lovastatin, simvastatin and pravastatin, fluvastatin, atorvastatin, and other statins), sequestrants (cholestyramine and colestipol), nicotinic acid, fenofibric acid derivatives (gemfibrozil, clofibrat, fenofibrate and benzafibrate), and probucol; anticholinergic agents such as muscarinic antagonists (ipratropium bromide); other compounds such as theophylline, sulfasalazine and aminosalicylates, e.g. 5-aminosalicylic acid and prodrugs thereof, antirheumatics.

Antiallergic drugs which are prone to be useful in combination with a compound of the present invention, 40-O-(2-hydroxyethyl)-rapamycin, e.g. prone to be useful according to the present invention, e.g. include antihistamines (H1-histamine antagonists), e.g. bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, fexofenadine, descarboethoxyloratadine, and non-steroidal anti- asthmatics such as β2-agonists (terbutaline, metaproterenol, fenoterol, isoetharine, albuterol, bitolterol, salmeterol and pirbuterol), theophylline, cromolyn sodium, atropine, ipratropium bromide, leukotriene antagonists (zafirlukast, montelukast, pranlukast, iralukast, pobilukast, SKB-106,203), leukotriene biosynthesis inhibitors (zileuton, BAY-1005); bronchodilators, antiasthmatics (mast cell stabilizers).

Anesthetics which are prone to be useful in combination with a compound of the present invention, 40-O-(2-hydroxyethyl)-rapamycin, e.g. prone to be useful according to the present invention, e.g. include e.g. include ethanol, bupivacaine, chloroprocaine, levobupivacaine, lidocaine, mepivacaine, procaine, ropivacaine, tetracaine, desflurane, isoflurane, ketamine, propofol, sevoflurane, codeine, fentanyl, hydromorphone, marcaine, meperidine, methadone, morphine, oxycodone, remifentanil, sufentanil, butorphanol, nalbuphine, tramadol, benzocaine, dibucaine, ethyl chloride, xylocaine, and phenazopyridine.
Antipruritics which are prone to be useful in combination with a compound of the present invention, 40-O-(2-hydroxyethyl)-rapamycin, e.g. prone to be useful according to the present invention, e.g. include menthol, camphor, oatmeal baths, pramoxine, calamine lotion, doxepin, chlorpheniramine, cyproheptadine, e.g. in the form of a hydrochloride, sulphapyridine, aluminum acetate (aluminum acetate), hydroxyzine e.g. in the form of a hydrochloride or in the form of a pamoate (VISTARIL®), fexofenadine, TRK-820, terfenadine, Burrow's solution, Unna's boot, tar emulsion.
Astringent agents which are prone to be useful in combination with a compound of the present invention, 40-O-(2-hydroxyethyl)-rapamycin, e.g. prone to be useful according to the present invention, e.g. include alum, oatmeal, witch hazel, very cold water, and rubbing alcohol eg Surgical Spirit; astringent preparations include silver nitrate, zinc oxide, zinc sulfate, Burow's solution, tincture of benzoin, vegetable substances such as tannic and gallic acids.

In each case where citations of patent applications or scientific publications are given, the subject-matter relating to the compounds comprises likewise the pharmaceutical acceptable salts thereof, the corresponding racemates, diastereoisomers, enantiomers, tautomers as well as the corresponding crystal modifications of above disclosed compounds where present, e. g. solvates, hydrates and polymorphs, which are disclosed therein. The compounds used as active ingredients in the combinations of the invention may be prepared and administered as described in the cited documents or in the product description, respectively. Also within the scope of this disclosure is the combination of more than two separate active ingredients as set forth above, i. e. a pharmaceutical combination within the scope of this disclosure could include three active ingredients or more. Further, both the first agent and the co-agent are not the identical ingredient.
The structure of the drug substances identified by code numbers, generic or trade names may be taken from the Internet, actual edition of the standard compendium "The Merck

Index" or from databases, e.g., Patents International, e.g., IMS World Publications, or the publications mentioned above and below.

Acitivity of compounds used in a method according to the present invention may be shown as follows:
MEF cell lines are generated in which there is loss of either Tsc1 or Tsc2 and which have a profound decrease in signaling through Akt. Decreased Akt activation may be seen in response to all stimuli, including serum, PDGF, EGF, insulin, and IGF1. The basis for decreased signaling in response to PDGF is pursued, and it is found that levels of PDGFRα and PDGFRβ are consistently decreased in Tsc1 or Tsc2 null cells, due to reduced transcription/translation. In addition, over-expression of PDGFRα by short or long-term transfection leads to increased Akt phosphorylation and stimulation in response to multiple growth factors. Further, stable expression of PDGFRα in Tsc2 null or Tsc1 null cell lines leads to enhanced tumor growth in vivo in the subcutaneous tumor model, indicating that this down-regulation is critical in determining the relatively benign nature of tumors occurring in TSC patients.

It may be also observed that signaling downstream of all of serum, PDGF, EGF, insulin, and IGF1 is also reduced in cells that are genetically engineered to lack both PDGFRα and PDGFRβ, indicating cross-talk among these receptors.

Signaling in Tsc 1 or Tsc 2 null MEFs in response to IGF1 in combination with a Compound A may be explored. Wortmannin may be used as an IGF1 inibtor.

Growth of Tsc1-/- and Tsc2-/- and control MEF cell lines in serum may be assessed in triplicate wells of cells plated at uniform density using the MTT assay. Growth responses are normalized individually for each cell line, to growth in the absence of any stimulation.

It can be demonstrated that there is a robust growth response to serum by both Tsc1 null and the control cell lines, but there is little growth response of these cells to IGF1 stimulation and that Compound A significantly reduces growth under all conditions, and blocks any growth response of the cells to IGF1.

Also trials of treatment with a Compound A in mouse models of Tsc may be carried out (1 month study). A first trial is performed in the Tsc1+/- mice, in which there is a sex effect on tumor development, so that female mice are studied only. A second trial is performed in Tsc2+/- mice, in which the use of the carcinogen ENU is performed to enhance and accelerate the rate of tumor formation in the kidney.

It can be demonstrated that Compound A has a dramatic effect in reducing kidney and liver tumors in Tsc1+/- mice and also in Tsc2+/- mice.

Activity in neurofibromatosis may be determined in NF1 deficient cell or animals (mice) models, e.g. analogously to TSC deficient cell or animals (mice) models. Compound A shows activity in corresponding assays.

## Claims

1. 40-O-(2-hydroxyethyl)-rapamycin for use in the treatment of disorders mediated via the Tuberous Sclerosis Complex, wherein the disorders are renal angiomyolipomas (AML), lymphangioleiomyomatosis (LAM), and/or subependymal giant cell astrocytomas (SEGAs); and/or for use in the treatment of symptoms associated with disorders mediated via the Tuberous Sclerosis Complex, wherein the symptoms are seizures; wherein 40-O-(2-hydroxyethyl)-rapamycin is administered orally in dosages from 2.5 mg up to 15 mg.

2. 40-O-(2-hydroxyethyl)-rapamycin for use in the treatment of disorders according to claim 1 or 40-O-(2-hydroxyethyl)-rapamycin for use in the treatment of symptoms according to claim 1, wherein 40-O-(2-hydroxyethyl)-rapamycin is administered in dosages from 2.5 mg up to 10 mg.

3. 40-O-(2-hydroxyethyl)-rapamycin for use in the treatment of disorders according to claim 1 or 40-O-(2-hydroxyethyl)-rapamycin for use in the treatment of symptoms according to claim 1, wherein 40-O-(2-hydroxyethyl)-rapamycin is administered in dosages 2.5 mg, 5 mg or 10 mg.

4. A pharmaceutical composition comprising 40-O-(2-hydroxyethyl)-rapamycin in association with at least one pharmaceutically acceptable excipient, for use in the treatment according to any one of claims 1 to 4.

## Patentansprüche

1. 40-O-(2-Hydroxyethyl)-Rapamycin zur Verwendung in der Behandlung von Krankheiten, die durch den TSC (Tuberous Sclerosis Complex) vermittelt werden, wobei es sich bei den Krankheiten um Nierenangiomyolipome (AML), Lymphangioleiomyomatose (LAM) und/oder subependymale Riesenzellenastrozytome (SEGAs) handelt; und/oder zur Verwendung in der Behandlung von Symptomen, die mit Krankheiten, die durch den TSC vermittelt werden, assoziiert sind, wobei es sich bei den Symptomen um Krampfanfälle handelt; wobei 40-O-(2-Hydroxyethyl)-Rapamycin oral in Dosierungen von 2,5 mg bis zu 15 mg verabreicht wird.

2. 40-O-(2-Hydroxyethyl)-Rapamycin zur Verwendung in der Behandlung von Krankheiten nach Anspruch 1 oder 40-O-(2-Hydroxyethyl)-Rapamycin zur Verwendung in der Behandlung von Symptomen nach Anspruch 1, wobei 40-O-(2-Hydroxyethyl)-Rapamycin in Dosierungen von 2,5 mg bis zu 10 mg verabreicht wird.

3. 40-O-(2-Hydroxyethyl)-Rapamycin zur Verwendung in der Behandlung von Krankheiten nach Anspruch 1 oder 40-O-(2-Hydroxyethyl)-Rapamycin zur Verwendung in der Behandlung von Symptomen nach Anspruch 1, wobei 40-O-(2-Hydroxyethyl)-Rapamycin in Dosierungen von 2,5 mg, 5 mg oder 10 mg verabreicht wird.

4. Pharmazeutische Zusammensetzung, umfassend 40-O-(2-Hydroxyethyl)-Rapamycin in Kombination mit mindestens einem pharmazeutisch unbedenklichen Grundstoff, zur Verwendung in der Behandlung nach einem der Ansprüche 1 bis 3.

## Revendications

1. 40-O-(2-hydroxyéthyl)-rapamycine pour utilisation dans le traitement de troubles médiés par l'intermédiaire du complexe de sclérose tubéreuse (TSC), les troubles étant des angiomyolipomes rénaux (AML), la lymphangioléiomyomatose (LAM) et/ou des astrocytomes sous-épendymaires à cellules géantes (SEGA) ; et/ou pour utilisation dans le traitement de symptômes associés à des troubles médiés par l'intermédiaire du complexe de sclérose tubéreuse, les symptômes étant des crises d'épilepsie ; la 40-O-(2-hydroxyéthyl)-rapamycine étant administrée par voie orale à des doses de 2,5 mg à 15 mg.

2. 40-O-(2-hydroxyéthyl)-rapamycine pour utilisation dans le traitement de troubles selon la revendication 1 ou 40-O-(2-hydroxyéthyl)-rapamycine pour utilisation dans le traitement de symptômes selon la revendication 1, la 40-O-(2-hydroxyéthyl)-rapamycine étant administrée à des doses de 2,5 mg à 10 mg.

3. 40-O-(2-hydroxyéthyl)-rapamycine pour utilisation dans le traitement de troubles selon la revendication 1 ou 40-O-(2-hydroxyéthyl)-rapamycine pour utilisation dans le traitement de symptômes selon la revendication 1, la 40-O-(2-hydroxyéthyl)-rapamycine étant administrée à des doses de 2,5 mg, 5 mg ou 10 mg.

4. Composition pharmaceutique comprenant de la 40-O-(2-hydroxyéthyl)-rapamycine en association avec au moins un excipient pharmaceutiquement acceptable, pour utilisation dans le traitement selon l'une quelconque des revendications 1 à 3.
